# EUROPEAN PATENT SPECIFICATION

(11) **EP 2 053 056 B1**
(45) Date of publication and mention of the grant of the patent: **26.12.2012**
(21) Application number: 07803672.0
(22) Date of filing: 01.08.2007
(51) Int. Cl.: C07K 14/09, A61K 39/135, A61P 31/14

(54) **DENDRIMERIC PEPTIDE CONSTRUCT FOR THE PREVENTION OF FOOT-AND-MOUTH DISEASE IN ANIMALS**
DENDRIMERES PEPTIDKONSTRUKT ZUR PRÄVENTION VON MAUL-UND-KLAUENSEUCHE BEI TIEREN
CONSTRUCTION PEPTIDIQUE DENDRIMÈRE POUR LA PRÉVENTION DE LA FIÈVRE APHTEUSE CHEZ LES ANIMAUX

(30) Priority: 02.08.2006 ES 200602142
(43) Date of publication of application: 29.04.2009
(73) Proprietor: Instituto Nacional de Investigación y Tecnología Agraria y Alimentaria (INIA), 28040 Madrid (ES); Consejo Superior de Investigaciones Cientificas (CSIC), 28006 Madrid (ES); Universitat Pompeu Fabra, 08002 Barcelona (ES)
(72) Inventor: BLANCO LAVILLA, Esther, 28110 Algete (Madrid) (ES); BÁRCENA DEL RIEGO, Juan, 28110 Algete (Madrid) (ES); CUBILLOS ZAPATA, Carolina, 28002 Madrid (ES); SOBRINO CASTELLÓ, Francisco, 28033 Madrid (ES); ANDREU MARTÍNEZ, David, 08006 Barcelona (ES); GARCÍA DE LA TORRE, Beatriz, 08004 Barcelona (ES)
(74) Representative: ZBM Patents
(86) International application number: PCT/ES2007/070146
(87) International publication number: WO 2008/015309

(56) References cited:
- WO-A1-99/66954
- FRANCIS M J ET AL: "Immunological evaluation of the multiple antigen peptide (MAP) system using the major immunogenic site of foot-and-mouth disease virus." IMMUNOLOGY JUL 1991 LNKD- PUBMED:1652552, vol. 73, no. 3, July 1991 (1991-07), pages 249-254, XP009136280 ISSN: 0019-2805
- SADLER K ET AL: "PEPTIDE DENDRIMERS: APPLICATIONS AND SYNTHESIS" JOURNAL OF BIOTECHNOLOGY, ELSEVIER SCIENCE PUBLISHERS, AMSTERDAM, NL, vol. 90, no. 3/04, 1 January 2002 (2002-01-01), pages 195-229, XP008020030 ISSN: 0168-1656
- TAM J P ET AL: "INCORPORATION OF T AND B EPITOPES OF THE CIRCUMSPOROZOITE IN A CHEMICALLY DEFINED SYNTHETIC VACCINE AGAINST MALARIA" THE JOURNAL OF EXPERIMENTAL MEDICINE, ROCKEFELLER UNIVERSITY PRESS, US LNKD- DOI:10.1084/JEM.171.1.299, vol. 171, no. 1, 1 January 1990 (1990-01-01), pages 299-306, XP000915591 ISSN: 0022-1007
- BLANCO E. ET AL.: 'Identification of T-cell epitopes in nonstructural proteins of foot-and-mouth disease virus' JOURNAL OF VIROLOGY vol. 75, no. 7, April 2001, pages 3164 - 3174, XP008103369
- VILLEN J. ET AL.: 'Towards a multi-site synthetic vaccine to foot-and-mouth disease: addition of discontinuous site peptide mimic increases the neutralization response in immunized animals' VACCINE vol. 22, no. 27-28, September 2004, pages 3523 - 3529, XP004526932
- DNG X. ET AL.: 'Immunological response of female macaques to the PH-20 sperm protein following injection of recombinant proteins or synthesized peptides' JOURNAL OF REPRODUCTIVE IMMUNOLOGY vol. 54, no. 1-2, March 2002, pages 93 - 115, XP008103372
- LU Y.-A. ET AL.: 'Chemically unambiguous peptide immunogen: preparation, orientation, and antigenicity of purified peptide conjugated to the multiple antigen peptide system' MOLECULAR IMMUNOLOGY vol. 28, no. 6, June 1991, pages 623 - 630, XP023682582
- DELIYANNIS G. ET AL.: 'Induction of long-term memory CD8+ T cells for recall of viral clearing responses against influenza virus' JOURNAL OF VIROLOGY vol. 76, no. 9, May 2002, pages 4212 - 4221, XP002434677
- NOVELLA I S ET AL: "Use of substituted and tandem-repeated peptides to probe the relevance of the highly conserved RGD tripeptide in the immune response against foot-and-mouth disease virus", FEBS LETTERS, ELSEVIER, AMSTERDAM, NL, vol. 330, no. 3, 20 September 1993 (1993-09-20), pages 253-259, XP025604034, ISSN: 0014-5793, DOI: 10.1016/0014-5793(93)80883-V [retrieved on 1993-09-20]
- PFAFF E ET AL: "Analysis of neutralizing epitopes on foot-and-mouth disease virus.", JOURNAL OF VIROLOGY JUN 1988 LNKD- PUBMED:2835507, vol. 62, no. 6, June 1988 (1988-06), pages 2033-2040, ISSN: 0022-538X

## Description

This invention is related to the field of veterinary medicine in general and specifically to the fields of immunology and chemical synthesis. In particular, the present invention refers to a dendrimeric peptide construct, to a process for its preparation and to the applications derived from its use as immunogen.

### BACKGROUND ART

The foot-and-mouth disease virus (also abbreviated as FMDV) is a picornavirus that produces a highly transmissible and devastating disease in farm animals, mainly cows, sheep and pigs.

In these animals, the virus enters through the epithelium (either through the mouth or the respiratory tract). It reaches the blood and causes fever during the first days, after which blisters appear in the mouth, snout, interdigital spaces, teats, mammary glands and areas where the skin is thin in general; the blisters burst leaving an aphtha or erosion that is later covered by the skin. The injuries or aphthae tend to be infected by bacteria, which makes very difficult to cure the animals and help them recover. Secondary infections can give rise to mastitis, hoof detachment, etc, which complicate and compromise the animal's survival. The illness on its own would not cause so many deaths, if there were not secondary infections.

The FMDV particle contains a positive-strand RNA molecule of approximately 8,500 nucleotides, enclosed within an icosahedral capsid comprising 60 copies of each of the four viral proteins, VP1-4. The genomic RNA encodes a single polyprotein from which the different viral proteins are generated, through a proteolytic process, due to the action of different viral proteases, which include nine mature non-structural proteins (NSPs). Each of these NSPs, as well as some of the precursor polypeptides, are involved in functions relevant to the life cycle multiplication of a virus in infected cells. FMDV shows a high genetic and antigenic variability, which is reflected in the existence of the seven serotypes and the numerous variants.

On the other hand, the antigenic structure of the FMDV, as recognised by B lymphocytes, has been characterised in detail. The main epitope B, which is considered to be the virus' main immunogen (against which most of the antibodies act, especially the neutralising antibodies), located on one of the defined structural motifs on the surface of the capsid, is continuous and immunodominant (Site A) and it is located in the GH loop, around positions 140 to 160 of protein VP1 (cf. Bittle J.L. et al., "Protection against foot-and-mouth disease by immunization with a chemically synthesized peptide predicted from the viral nucleotide sequence", Nature 1982, vol. 298, pgs 30-33). This epitope has been widely used as an immunogen in various studies (cf. Brown F., "New approaches to vaccination against foot-and-mouth disease", Vaccine 1992, vol. 10(14), pgs 1022-1026). In 1986, DiMarchi and colleagues (cf. DiMarchi et al., "Protection of cattle against foot-and-mouth disease by synthetic peptides", Science 1986, vol. 232, pgs. 639-641) reported protection against viral challenge infection in cattle immunised with a peptide in which the VP1 residues 140-160 were colinearly synthesised with the residues corresponding to positions 200-213 of the same protein. However, further studies involving a significant number of animals have pointed out that this peptide epitope provides limited protection in natural FMDV hosts (cf. Barteling S.J, "Possibilities and limitations of synthetic peptide vaccines", Adv. Biotechnol. Processes. 1988, vol. 10, pgs. 25-60).

Furthermore, several T-cell epitopes recognised by lymphocytes from FMDV infected pigs have been identified in non-structural proteins (cf. Blanco E. et al., "Immunodominant T Cell epitopes in non-structural polypeptides of Foot-and-mouth Disease Virus", Journal of Virology 2001, vol. 75, pgs. 3164-3174). Among these epitopes, epitope T of protein 3A [21-35], synthesised colinearly with epitope B of VP1 [137-156], was able to efficiently stimulate lymphocytes from an infected animal and induced significant levels of serotype-specific anti-FMDV activity in vitro.

On one hand, the problem with the foot-and-mouth disease virus, which has prompted such health concerns, is that it is very contagious. The infected animals release the virus through their saliva (which is produced in abundance as a result of the disease), their urine and faeces: when the blisters burst, it is also released the virus from inside. Milk and meat contain the virus, too. This means that anything which is contact with the infected animal will be easily contaminated: floors, tools, vehicles, meat, etc. Likewise, the virus is very resistant under particular conditions. All of this means that the disease can very easily be transmitted to the people handling the animals. Therefore, it is necessary to take strict precautionary measures to avoid the contagion and spread of this illness which, although not being dangerous for human health, can cause severe economic losses.

Currently, foot-and-mouth disease is mainly controlled through the use of vaccines based on the inactivated virus. Both FMDV infections and the immunisation based on these vaccines usually elicit high levels of specific circulating antibodies that are correlated with the protection from the related homologous virus and antigenically related virus.

Francis M. J. et al., discloses a FMDV vaccine comprising the VP1 fragment 141-160 in a tetrameric MAP structure (cf. Francis M J et al., "Immunological evaluation of the multiple antigen peptide (MAP) system using the major immunogenic site of foot-and-mouth disease virus", Immunology 1991, vol. 73, pgs. 249-254). A serological assay of said vaccine was performed using guinea-pigs inoculated with the FMDV O₁ Kaufbeuren virus.

Sadler K. et al., reviews the different types of peptide dendrimers and the synthetic methods commonly employed to generate them (cf. Sadler K. et al., "Peptide dendrimers: applications and synthesis", Journal of Biotechnology 2002, vol. 90, pgs. 195-229).

Tam J. P. et al., discloses the effect of the stoichiometry, orientation and arrangement of the B and T epitopes on the immunogenicity of the MAPs(Tam J. P. et al., "Incorporation of T and B epitopes of the circumsporozoite protein in a chemically defined synthetic vaccine against malaria", J. Exp. Med. 1990, vol. 171, pgs 299-306).

On the other hand, despite the progress in recent years, the vaccines known and used until now show several disavantatges, such as the requirement of a cold chain to preserve their stability, the need for periodic revaccinations, the risk of infectious virus release during its production, and its non-identifying nature, in other words, the difficulty of serologically distinguishing infected animals from those that have been vaccinated.

These limitations have led us to search for alternative and safe immunogens.

### DESCRIPTION OF THE INVENTION

The inventors of the present invention have designed a dendrimeric peptide construct which, when administrated to animals, and particularly to pigs, elicits an immune system response which is effective, providing total protection against the foot-and-mouth disease virus.

Thus, in a first aspect the present invention provides a dendrimeric peptide construct of formula (I):

(X-B)n-L-T (I)

wherein
T is a peptide comprising the SEQ ID NO: 1 sequence
B is a peptide comprising the SEQ. ID NO: 2 sequence
X is an acyl group which is bound to the N-terminal end of peptide B;
n is 3 or 4; and
L corresponds to a lysine core of formula (II) 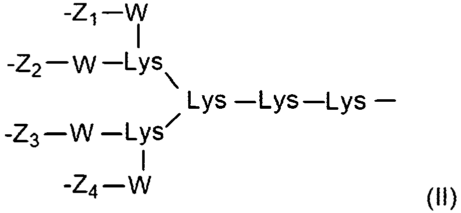 wherein
W is a linker group;
Z₁-Z₄ are identical or different to each other and are selected from the group consisting of cysteine and -OH, provided that at least three of
the Z₁ to Z₄ groups are cysteine;
the peptide B being bound to the lysine core by its C-terminal end and the peptide T being bound to said lysine core by its N-terminal end.

The SEQ ID NO: 1 sequence corresponds to the epitope T of the non-structural protein 3A (residues 21-35) of the FMDV.

The SEQ ID NO: 2 sequence corresponds to the epitope B of the capsid VP1 protein (residues 136-153) of a FMDV C-S8 isolate of serotype C, also called antigenic site A.

In the present invention, by "linker group" it is understood a bi-functional unit that makes possible the linking of peptide B to the lysine core (through Z₁-Z₄), through a thioether covalent type bond, during the synthesis of the dendrimeric peptide construct.

Proteins VP1 and 3A of the foot-and-mouth disease virus have been extensively disclosed in the prior art, as well as epitopes T and B (SEQ ID NO: 1 and SEQ ID NO: 2, respectively), which are part of the construct of the construct of the present invention, along with the fragments or derivatives thereof.

According to the present invention, the dendrimeric peptide construct is characterised by having a central core with three branched lysine residues. This core elongates in the C-terminal direction with two additional lysine residues, which define a putative cleavage site for cathepsin type proteases, followed by the peptide T sequence (which comprises the peptide T sequence of the FMDV defined by Ala21-Lys35 residues of protein 3A or a fragment thereof). The four branches defined by the lysine residues of the core are connected, through a linker group, to cysteine residues or a -OH group. In this way, the branches that bind a cysteine will bind, at the same time, one copy of peptide B (which comprises the immunodominant epitope B sequence of FMDV, defined by residues Tyr136-Arg153 of VP1, or a fragment thereof), which is acylated at its N-terminal end.

In another embodiment of the first aspect of the invention, T is a peptide of sequence SEQ ID NO: 1 and B is a peptide of sequence SEQ ID NO: 2.

In another embodiment of the first aspect of the invention, T is a peptide T is a peptide that comprises the SEQ ID NO:1 sequence, and B is a peptide that comprises the SEQ ID NO:5 sequence.

In fact, SEQ ID NO: 5, which includes the fragment of SEQ ID NO: 2 mentioned above, corresponds to the epitope B of the capsid VP1 protein (residues 137-155) of a FMDV isolate of serotype O (O PanAsia). This ID NO: 5 sequence is functionally identical to ID NO: 2 sequence of serotype C.

In yet another embodiment of the first aspect of the invention, X is acetyl (abbreviated as Ac).

In still yet another embodiment of the first aspect of the invention, W is a group -(CH₂)ₙ-CO-, wherein n is a number between 1 and 3. Preferably W is -CH₂CO-.

In another embodiment of the first aspect of the invention L represents: 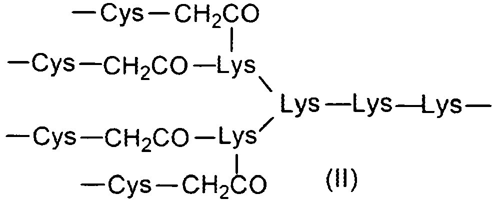 wherein each cysteine is bound to the CH₂CO linker group through its side chain by a thioether bond.

In yet another preferred embodiment of the first aspect of the invention, the dendrimeric peptide construct has the following structure (III) 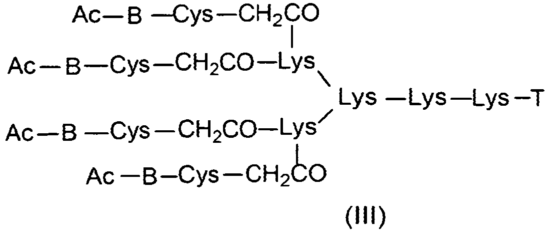 wherein
T is a peptide of SEQ ID NO: 1 sequence;
B is a peptide of SEQ ID NO: 2 sequence; and
X represents acetyl (abbreviated as Ac).

The present invention also encompasses more complex structures in which various constructs, in accordance with the first aspect of the invention, are chained together.

In a second aspect the present invention refers to a process for preparing a dendrimeric construct as defined in the first aspect of the invention, which comprises the ligation reaction of a peptide of formula (IV): 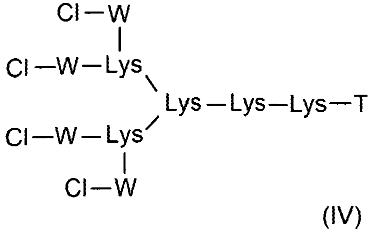 with a peptide (V),

X-B-Cys (V)

wherein
Cl is chlorine and
T, X, W and B are as defined above;
and wherein the ligation reaction is carried out in the presence of a chaotropic agent and at a pH between 8 and 9.

In a preferred embodiment of the second aspect of the invention, the ligation reaction is carried out at a pH of 8.5.

In another embodiment of the second aspect of the invention, the chaotropic agent is guanidine hydrochloride. The presence of the chaotropic agent in the ligation reaction minimises the possible self-aggregation of dendrimer chains under growing.

In yet another embodiment of the second aspect of the invention, the peptide of formula (V) is in a molar excess comprised between 3 and 5 in respect of the peptide in formula (IV).

As mentioned above, the inventors of the present invention have found that the dendrimeric peptide construct has immunogenic properties.

In fact, as illustrated below, it has been found that, in comparison with other immunogens described in the prior art, the construct of the present invention has improved activity, in such a way that it effectively prevents the infection elicited by the foot-and-mouth disease virus and, in addition, it is identifiable, in other words, it is possible to serologically distinguish the infected animals from those that have been vaccinated. Proof of this is that the determination of antibodies against the non-structural protein 3ABC was negative in the immunised and challenged pigs by the virus, whereas in the controls, the anti-3ABC response was positive, as described below. This seroconversion of antibodies against the 3ABC protein is one of the most widely used parameters for identifying animals infected with FMDV, including asymptomatic carriers of the infection (cows and sheep, among others). This is extraordinarily important, because after performing emergency vaccinations, it would not be necessary to slaughter any of the vaccinated animals, as they would be clearly distinguishable from the infected ones.

In addition, the construct of this invention provides total protection against the virus. In this sense, it should be noted that, unlike other constructs, it has been found that this construct stimulates the secretion of IgA, which can be found on the swabs used to take mucosal tissue samples from the animals that have been administered the construct. In particular, it has been confirmed that the dendrimeric peptide construct of the invention induces high titres of specific antibodies against the FMDV (IgGs) in serum, as well as specific antibodies against the FMDV (IgA) in serum and in nasal fluids. The prophylactic effect that characterises the construct of the invention is also due to the fact that it induces lymphocytes T which are able to proliferate specifically against the FMDV. This would explain the prophylactic effect that characterises the dendrimeric construct of the present invention.

In view of all the foregoing, this invention refers in a third aspect to a dendrimeric peptide construct in accordance with the first aspect of the invention for use as immunogen.

In a fourth aspect, the present invention refers to a veterinary composition that comprises an effective amount of at least one dendrimeric peptide construct in accordance with the first aspect of the invention.

In a preferred embodiment of the fourth aspect of the invention the composition comprises adequate amounts of at least one adjuvant for its administration in the form of a vaccine.

The adjuvants that can be used in the preparation of a vaccine in accordance with the invention are well-known to those skilled in the art (cf. McKercher PD et al., "A review of the current status of oil adjuvants in foot-and-mouth disease", Dev. Biol. Stand. 1976, vol. 35, pgs. 107-112; Dong XN et al., "Candidate multi-peptide-vaccine against classical swine fever virus induced potent immunity with serological marker", Vaccine 2005, vol. 25, pgs. 3630-3633). Illustrative non-limitative examples of adjuvants include Freund's complete and incomplete adjuvant.

In a fifth aspect the present invention refers to the the construct in accordance to the first aspect of the invention for preventing the foot-and-mouth disease. This aspect can be formulated as the use of a construct in accordance with the first aspect of the invention for the manufacture of a medicament for the prevention of foot-and-mouth disease in an animal. Preferably, the animal is a pig.

The invention also refers to a method for preventing foot-and-mouth disease in an animal, comprising administering an effective amount of at least one construct in accordance with the first aspect of the invention.

Unless explicitly defined otherwise, all the technical and scientific terms used herein have the same meaning as they would as commonly understood by an expert in the field to which this invention belongs. Throughout the descriptions and claims, the word "comprises" and its variants are not intended to exclude any other possible technical characteristics, additives, components or steps. Additional objects, advantages and features of the invention will become apparent to those skilled in the art upon examination of the description or may be learned by practice of the invention. The following examples and drawings are provided by way of illustration, and is not intended to be limiting of the present invention.

### BRIEF DESCRIPTION OF THE DRAWINGS

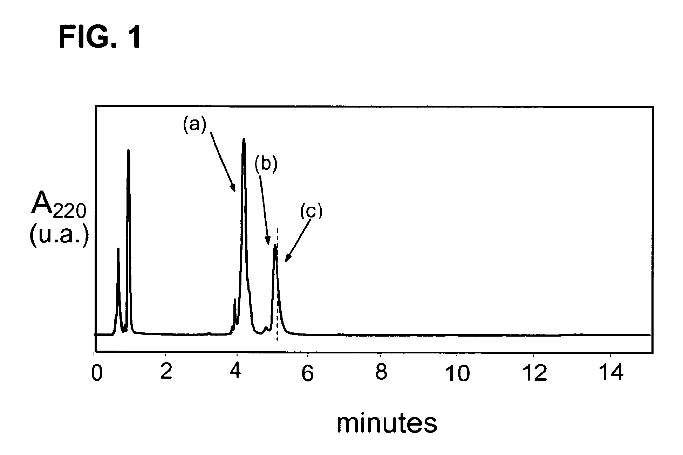

FIG. 1 corresponds to a MALDI-TOF mass spectrometry of samples collected after 72 hours from the beginning of the ligation reaction between the tetravalent epitope T (chloroacetylated) and the epitope B-cell peptide. The larger area of the later-eluting peak shows the progressive enrichment of the conjugate in species with 3 and 4 copies of epitope B, concentrated in the front fractions of the peak, on the left of the dashed line. (a) peak indicating the epitope B peptide; (b) peak indicating the dendrimeric peptide construct with 3 and 4 epitope B peptides; (c) peak indicating the dendrimeric peptide construct with 2 and 3 epitope B peptides.

### EXAMPLES

### 1. Obtaining a dendrimeric peptide construct of the invention

### A) Obtaining the tetravalent T-epitope peptide.

Linear sequence SEQ ID NO: 3
Lys-Lys-Ala-Ala-Ile-Glu-Phe-Phe-Glu-Gly-Met-Val-His-Asp-Ser-Ile-NH2
was assembled as a C-terminal carboxamide in a polystyrene resin -1%-divinylbenzene functionalised with a Rink amida-type linker, using Fmoc chemistry in a ABI433 automated synthesiser (Applied Biosystems, Foster City, CA), running the FastMoc synthesis programme.

For the side chains of the tri-functional amino acids, the protective groups tert-butoxycarbonyl (Lys), tert-butyl (Ser, Asp, Glu) and trityl (His) were used. Couplings were performed with 10 equivalents (1 mmol) of Fmoc-amino acid, 2-(H-benzotriazol-1-yl)-1,1,3,3-tetramethylurone hexafluorophosphate (HTBU), and N-hydroxybenzotriazole (HOBt), in the presence of 20 equivalents of N,N-diisopropylethylamine, all with N,N-dimethylformamide (DMF) as solvent. Recouplings were introduced for the last five residues of the synthetic sequence (Ile to Lys). The peptidyl-resin was deprotected (20% piperidine in DMF, 2 + 20 min) and 5 equivalents of Fmoc-Lys(Fmoc)- OH were manually coupled using the activation with diisopropylcarbodiimide and HOBt. After Fmoc removal, a double coupling was performed with 10 equivalents of Fmoc-Lys (Fmoc)-OH using the same type of activation. After Fmoc deprotection (as above), a coupling was made with 24 equivalents (6 equivalents for each one of the 4 generated amino groups) of the pentachlorophenyl ester of chloroacetic acid, in DMF. Subsequently, the peptide was deprotected and cleaved from its polymeric support by treatment with trifluoroacetic acid-triisopropylsilane-water (96:2:2 v/v, 2 h, 25 °C). The crude peptide was precipitated from the solution by diluting it with 5 volumes of chilled methyl tert-butyl ether, followed by centrifugation at 4000 rpm and 5 °C for 10 min. The residue was reconstituted in aqueous acetic acid at 10% and was freeze-dried.

The product was analysed by reverse phase HPLC in a Shimadzu LC- 2010A system equipped with a Phenomenex Luna C8 column (3 µm, 0.46 x 5 cm), using a linear gradient (10-40%) of buffer B in buffer A for 15 minutes at a flow rate of 1 mL/min, buffer A being trifluoroacetic acid in water at 0.045%, and B the same acid in acetonitrile at 0.036%. The product was purified by preparative scale in a Shimadzu LC-8A system using a Phenomenex Luna C8 column (10 µm, 2.1 x 25 cm) eluted with the gradient indicated above at a flow rate of 25 mL/min during 60 minutes. The fractions containing the product with adequate purity were combined and lyophilised, subsequently being characterised by MALDI-TOF mass spectrometry in a Voyager DESTR instrument (Applied Biosystems, Foster City, CA) using the α-cyano-4'-hydroxycinnamic acid as a matrix. A peak was obtained with m/z 2637.4, compatible with the target structure (MH+, monoisotopic).

### B) Obtaining the epitope B peptide.

Sequence SEQ ID NO: 4 Ac-Tyr-Thr-Ala-Ser-Ala-Arg-Gly-Asp-Leu-Ala-His-Leu-Thr-Thr-Thr-His-Ala-Arg-His-Cys-NH₂
was assembled by solid phase synthesis methods, using Fmoc chemistry in an automated ABI 433A synthesiser and the same synthesis programs described in the previous section. The same side chain protector groups were used, including additionally tert-butyl for Tyr and Thr, and 2,2,4,6,7-pentamethyldihydrobenzofuran-5-sulphonyl for Arg. The deprotection and cleavage of the peptydil-resin were carried out through a treatment with trifluoroacetic acid, triisopropylsilane, ethanedithiol and water (94:2:2:2 v/v, 2 h, 25 °C), with subsequent processing identical to that described in the previous section. The purified product gave m/z 2222.3 in its analysis by MALDI-TOF mass spectrometry.

### C) Conjugation of T and B epitope peptides.

8 mg of the peptide corresponding to epitope T, being its N-terminal end extension tetravalent and chloroacetylated, were dissolved in 30 mL of 20 mM Tris buffer, pH 8.5, containing 2.5 mM guanidine chloride. The peptide corresponding to epitope B (90 mg) was added in seven portions to said solution during a period of 4 h, controlling the solution's pH and correcting it by adding NaOH 0.001 M solution in such a way as to maintain the pH within the 8-8.5 range.

At regular intervals, aliquots of the reaction were taken and analysed by HPLC using a linear gradient (5-95%) of buffer B in A for 15 min (buffers A and B defined in section A above). The composition of the newly formed conjugate peaks was determined by MALDI-TOF mass spectrometry.

After 36 h of reaction at 25°C, and when a substantial part of the epitope B peptide was dimerized, 2 mg of tris-carboxyethylphosphine were added to the reaction mixture, readjusting again the pH to 8.5 and allowing the reaction to continue until 72 h, when no further changes in the HPLC profile could be observed. At this point the reaction mixture was acidified with a few drops of trifluoroacetic acid, concentrated by partial lyophilisation and purified by preparative HPLC as described previously. The front fractions of the late-eluting peak were analysed one by one by MALDI-TOF mass spectrometry, confirming that they corresponded to conjugates with three and four copies of the epitope B peptide (see FIG. 1). The fractions richest in four copy conjugates were selected, which were then combined and lyophilised prior to their use as immunogens.

### 2. Virus

The FMDV strain C-S8 (Sta.Pau/70) (ceded by the laboratory of Dr. Esteban Domingo (CBM-CSIC)) was grown in monolayers of BHK cells (access no. ATCC: CCL10). The batch of virus used in the inoculations corresponds to a second passage in cells, and the one used in the lymphoproliferation tests to a fourth passage.

To this effect, the different preparations of the FMDV were grown by infection of confluent monolayers of BHK-21 cells. Following a period of adsorption (1 hour at 37°C), Eagle medium modified by Dulbecco (Dulbecco and Freeman, 1959) (DMEM, Bio-Whittaker) was added, supplemented with 5-10% bovine fetal serum (BFS) (Bio-Whittaker) and using as antibiotics penicillin (10 U/ml) and streptomicine (0.1 mg/ml) (Gibco), and incubation continued at 37°C until achieving a cytopathic effect of 90% (generally within 24-48 hours). At that moment the infection supernatants were collected, treated with 2% of chlorophorm, centrifuged for 10 minutes at 3,000 x g, at 4°C. The clarified supernatants were kept at -70°C until the moment of being used. Successive passages were carried out inoculating again cellular monolayers with the collected supernatants.

### 3. Vaccination and infection of swine

Landrance x Large White (n=4) pigs, of 3-4 months old were immunised intramuscularly with 1.4 mg of the TB dendrimeric peptide construct, emulsified with Freund's complete (first dose) or incomplete (second dose) adjuvant, mixing equivalent volumes of the construct and adjuvant in a final volume of 1 ml.

Eighteen days after administering the second dose of the dendrimeric peptide construct, these pigs were inoculated intradermally, (inoculation in coronary band), with 10⁴ UFP of the type C FMDV, C-S8 cl1 isolate (Sta.Pau/70).

Blood and sera samples were collected on days 0 (moment when the first dose of the dendrimeric peptide construct was administered), 14 (from the first dose of the vaccine), 21 (a second dose of vaccine was administered) and 39 (the virus was administered). After inoculation with the FMDV, in addition to these samples nasal and pharyngeal swabs were collected also on days 0, 3, 7 and 10 post-inoculation.

All the animals were slaughtered at 10 days post-inoculation. Three of these pigs did not develop the lesions typical of FMDV infection, during the 10 days following inoculation that they were monitored. In the fourth pig (pig no. 3), only a small sore was observed on the mouth, during day 4 post-inoculation, a lesion that was resolved within 24 h.

With the aim of controlling the challenge with the virus, two control pigs were included in the experiment. Pigs nos. 5 and 6 were kept in direct contact with the 4 immunised and challenged animals. Blood and sera samples and nasal and pharyngeal swabs were collected on days 0, 3, 7 and 10 post-contact with the inoculated pigs. On day 14 both pigs were inoculated by intradermally with the same titer and the same batch of virus as used to challenge the immunised pigs. Again, blood and sera samples, and nasal and pharyngeal swabs were collected on days 0, 3, 7 and 10 post-inoculation. Both animals developed the typical lesions of infection with FMDV within 3-4 days post-inoculation.

All the animals employed in this study were, initially, free of infection with FMDV, including the control pigs, as confirmed by the absence of specific antibodies against FMDV at time 0. Following the challenge, those immunised with the dendrimeric peptide construct continued to be free of infection while the control pigs became infected and developed the disease.

### 4. Specific humoral response against FMDV

The specific humoral response against the complete virus was determined by analysing the sera of pigs number 1 to 6 in 1/200 dilution through a capture ELISA.

To this effect, 96-well ELISA plates (Maxisorp-Nunc) were coated with anti-FMDV rabbit serum C1-Noville strain (supplied by the worldwise reference laboratory for FMDV, Pirbright, GB), overnight at 4 °C. The plates were washed with PBST (PBS with 0.05% of Tween20) and blocked for 1 hour at 37°C, adding 50 µl/well of PBSTM (PBST with 5% of skimmed milk). After washing, 50 µl of the antigen (CS8cl1 virus) was added to each well of the ELISA plate and it was incubated at 37°C during 1 hour.

Two-fold dilutions of each one of the serum from the pigs (numbers 1 to 6) were analysed, incubating them during 1 hour at 37 °C. The plates were washed again and were incubated for 1 further hour at 37 °C in the presence of the protein-A conjugate labelled with peroxidase (Sigma). Following a final wash, the substrate, the OPD (Orthophenylendiamine, Sigma), for the used peroxidase was added, having previously added H₂O₂. The reaction was stopped after 10 minutes with H₂SO₄ 3N, and an automatic reader at 492 nm was read. The antibodies titre is expressed as the dilution of serum at which OD₄₉₂ is more than 50% of that obtained in the negative serum control.

The following table includes the values obtained on analysing the sera collected at days 0, 14 (14 days after the first immunisation), 21 (21 days after the first immunisation and moment when a second dose of the vaccine is administered), 39 (18 days post-2^{nd} immunisation and moment at which the virus is administered) and 49 (10 days post-inoculation).

**TABLE 1**

| Pigs immunised with peptide | 0 days | 14 days | | 21 days | | 39 days | | 49 days |
|---|---|---|---|---|---|---|---|---|
| No. 1 | 0.23 | 0.484 | | 0.947 | | 1.585 | | 1.481 |
| No. 2 | 0.25 | 0.414 | | 1.092 | | 1.46 | | 1.384 |
| No. 3 | 0.22 | 0.319 | | 0.458 | | 1.344 | | 1.389 |
| No. 4 | 0.021 | 0.344 | | 0.847 | | 1.553 | | 1.573 |

| Control pigs | 0 days | | 10 dpc | | | | 10 dpi | |
|---|---|---|---|---|---|---|---|---|
| No. 5 | 0.2 | | 0.305 | | | | 1.73 | |
| No. 6 | 0.23 | | 0.251 | | | | 1.61 | |

The antibodies titres against FMDV in the pigs immunised with the TB dendrimeric peptide construct were significant and high 18 days post-immunisation of the second dose.

In particular, the antibodies titres before and after the viral challenge were similar, around 1.5 Iog10. However, neither of the two control pigs (pigs nos. 5 and 6) developed a significant antibodies response at times 0 and 10 days post-contact, but did at 10 days post-inoculation. At this time the maximum titres reached were similar to those observed in the immunised and challenged pigs.

The results obtained indicated that the dendrimeric peptide construct was capable of inducing the production of antibodies that recognised FMDV. The titres obtained were high and, following the challenge they did not increase, which suggested that the animals were protected since no increase was detected in the antibodies titre following inoculation of FMDV.

### 5. Response of specific IgA antibodies against FMDV

The detection of IgA antibodies was determined through indirect ELISA, analysing sera and nasal and pharyngeal swabs collected at times 0, 14, 21 and 39 days post-immunisation and 3, 7 and 10 days post-inoculation with the FMDV. In order to carry out this ELISA, the procedure described in point 4 for the ELISA-FMDV was followed, except that instead of using the A-HRPO protein conjugate, an anti-IgA pig monoclonal antibody labelled with HRPO was used, diluted at 1/1000 (Serotec). The titre of IgA was calculated as the log of the serum dilution that provides an OD₄₉₂ signal equal to 1.

The results obtained were as follows:

**TABLE 2**

| Pigs immunised with peptide | 0 days | 14 days | | 21 days | | 39 days | | 49 days |
|---|---|---|---|---|---|---|---|---|
| No. 1 | 0.0 | 0.0 | | 2.2 | | 5.0 | | 4.0 |
| No. 2 | 0.0 | 0.0 | | 2.7 | | 4.6 | | 3.7 |
| No. 3 | 0.0 | 0.0 | | 0 | | 3.4 | | 3.4 |
| No. 4 | 0.0 | 0.0 | | 2.2 | | 3.5 | | 3.6 |

| Control pigs | 0 days | | 10 dpc | | 10 dpi | | 24 dpi | |
|---|---|---|---|---|---|---|---|---|
| No. 5 | 0.0 | | 0.0 | | 3.5 | | 4.3 | |
| No. 6 | 0.0 | | 0.0 | | 2.7 | | 4.5 | |

The maximum peak of IgA response was detected at 39 days post-immunisation. These maximum titres were very similar to those obtained 10-days post-inoculation (day 49), which suggests a possible implication of this immune response mechanism in the protective response against FMDV. The IgA titre determined in the serum of the two control pigs at 10 days post-inoculation was about 2 and 3, significantly lower than the one reached in the pigs immunised with the dendrimeric peptide construct before the challenge. Specific IgAs were also detected in the nasal swabs of the immunised pigs, although not in the pharyngeal swabs.

The fact of finding IgA in nasal swabs suggested that the stimulation of the mucose response was especially focused here and not so much in the pharynx, although these differences could result from the greater difficulty in taking pharyngeal swabs compared to nasal swabs.

With this experiment the immunogenic ability of the dendrimeric peptide construct was confirmed since high seric levels of IgA were detected.

### 6. Determination of neutralising antibodies

The neutralising activity of FMDV was determined in vitro in the sera of the immunised pigs, collected at time 0 and 39 days post-immunisation, and in the sera of the control pigs, collected at days 10 post-contact and 10 post-inoculation.

The test consisted of a standard neutralisation test, carried out on 96-well plates, incubating equivalent volumes of two-fold diluted serum, with 100 infective doses 50 (unit in which infective viruses tend to be measured, infective doses 50 are those that infect approximately 50% of the inoculated cells) of the FMDV- CS8cl1. This serum-virus mixture was incubated for 1 hour at 37 °C, after which the neutralising activity was determined by adding BHK-21 cells (access no. ATCC: CCL10). The neutralisation titres are expressed as the log₁₀ of the serum dilution that provides a neutralisation of 50% of the inocula of the virus used.

**TABLE 3**

| Pigs immunised with peptide | 39 days post-immunisation | 10 days post-inoculation |
|---|---|---|
| No. 1 | 2.2 | 2.5 |
| No. 2 | 1.9 | 2.2 |
| No. 3 | 1.9 | 2.2 |
| No. 4 | 2.2 | 2.2 |

| Control pigs | 10 days post-contact | 10 days post-inoculation |
|---|---|---|
| No. 5 | <1.3 | 2.8 |
| No. 6 | <1.3 | 3.4 |

The titre of neutralising antibodies before the viral challenge in the pigs immunised with the dendrimeric peptide construct was about 1.9 -2.2 log10. Following the challenge, these titres only increased slightly.

The fact that no significant increases were obtained, was a further indicative proof that the animals were already stimulated and protected since with the challenge the immune parameters did not increase.

### 7. Lymphoproliferation test

This assay was carried out as described in Blanco and colleagues (cfr. Blanco E. et al., "An interspecies MHC-restricted Th cell epitope on the capsid protein VP4 of foot-and-mouth disease virus", Journal of Virology 2000, vol. 74, pp. 4902-4907).

Blood samples were collected from pigs nos. 1 to 4 in a 5 µM EDTA buffer and were immediately used to prepare the mononuclear cells of peripheral blood (abbreviated as MCPB). This assay was carried out on round-bottomed 96-well plates (Nunc). Briefly, 2.5 x 10⁵ of MCPB per well were incubated, in triplicate, in a final volume of 200 µl, in complete cultivation medium RPMI (SFB 10% (v/v), 2-mercaptoethanol 50µM), in the presence of various concentrations of:
i) FMDV ranging from 4 x 10³ to 2 x 10⁶ UFP,
ii) Synthetic peptides ranging from 100 µg/ml to 0.03 µg/ml. As synthetic peptide the epitope T (SEQ ID NO: 1), the epitope B (SEQ ID NO:2), and the dendrimeric peptide construct of the invention, was used;
iii) Controls of non-stimulated MCPB.

The plates were incubated at 37 °C in an atmosphere of 5% CO₂ for 4 days. Next, 0.5 µCi of methyl- [3H] thymidine was added to each well was incubated for further 18 h at 37 °C. The cells were collected using a cell harvester and the incorporation of radioactivity to the DNA was determined by liquid scintillation and reading in a Microbeta counter (Pharmacia) radioactivity counter.

The results are expressed in SI (Stimulation index = counts per minute of stimulated cells / average counts per minute of unstimulated cells):

**TABLE 4**

| Sl | Pig No. 1 | Pig No. 2 | Pig No. 3 | Pig No. 4 |
|---|---|---|---|---|
| B | 26 | 29.5 | 11 | 17.7 |
| T | 7.7 | 1.3 | 1.7 | 4.5 |
| TB | 21.4 | 30.4 | 12.4 | 24 |
| Virus | 6.6 | 32.2 | 20.1 | 31.3 |

The obtained SI values were significant, which confirmed that the dendrimeric peptide construct used was capable of priming lymphocytes in vivo which were capable of recognising in vitro the epitopes present in said construct, and even in the context of a particular native viral. Definitively, the dendrimeric peptide construct also stimulated the porcine cellular response.

In order to analyse the role of the Major Histocompatibility Complex (abbreviated as MHC) in the lymphoproliferative response, the following anti-swine MHC monocolonal antibodies were used (SLA): 74-11-10 (IgG2β) anti-SLA class I (cfr. Pescovitz et al., "Effect of class II antigen matching on renal allograft survival in miniature swine", J. Exp. Med. 1984, vol. 160, pp. 1495-1508) and MSA-3 (IgG2α) anti-SLA class II (cfr. Hammerberg C. et al., "Characterization of monoclonal antibodies directed against swine leukocytes", Vet. Immunol. Immunopathol. 1986, vol. 11, pp. 107-121). A total of 15 µl of the corresponding monoclonal antibody (1 mg/ml) was added per well at the beginning of the culture and additional 15 µl were added following 24 hours of incubation. The plates were processed in the same way as indicated above. The concentration of the anti-MHC class II monoclonal used inhibits the stimulation with ConA but not with the PHA ionophore, while the class I monoclonal does not affect such proliferations (cfr. Bullido et al., "Characterization of five monoclonal antibodies specific for swine class II major histocompatibility antigens and crossreactivity studies with leukocytes of domestic animals", Dev. Comp. Immunol. 1997, vol. 21, pp. 311-322). Under the experimental conditions used, the observed lymphoproliferative response was not significantly inhibited by the anti-SWC3 monoclonals (monocytes marker) or anti-γδTCR (cfr. Blanco et al., "An interspecies MHC-restricted Th cell epitope on the capsid protein VP4 of foot-and-mouth disease virus", Journal of Virology 2000, vol. 74, pp. 4902-4907).

The lymphoproliferative response against FMDV and to the TB dendrimeric peptide construct was significant and high in the immunised pigs, before the viral challenge, confirming that the dendrimeric peptide construct stimulated the porcine cellular response.

### 8. Detection of antibodies against the non-structural 3ABC protein of the FMDV

In order to determine the antibodies against the 3ABC protein, an indirect ELISA was used, based on using as antigen the 3ABC recombinant protein expressed in E. coli and purified as described by Bergmann and colleagues (cfr. Bergmann IE et al., "Diagnosis of persistent aphtovirus infection and its differentiation from vaccination response in cattle by use of enzyme-linked immunoelectrotransfer blot analysis with bioengineered nonstructural viral antigens" Am. J. Vet. Res. 1993, vol. 54(6), pp. 825-831). The procedure followed to carry out this ELISA was described by Blanco E. and colleagues (cfr. Blanco E. et al.,"Serological evidence of subclinical FMD infection in sheep population during the 1999 epidemic in Morocco", Veterinary Microbiology 2002, vol. 85/1, pp. 13-21).

Following procedures described in the preceding sections, the sera were analysed by ELISA-3ABC before and after the viral challenge of the animals immunised with the dendrimeric peptide construct (pigs nos. 1 to 4) and of the two control pigs (pigs nos. 5 and 6).

The ELISA-3ABC used is based on the one described by the PANAFTOSA laboratory and that has been considered as the reference test for the discrimination between infected and vaccined animals by the World Animal Health Organisation (International Office of Epizooties, Chapter 2: Foot and mouth disease, in "Manual of standards for diagnostic tests and vaccines", 5th Edition, Ed. OIE Standards Commission).

While the sera of the two controls were positive to 3ABC from 10 dpi, none of the four immunised pigs showed significant antibody titres against this protein (signals <0.3), as reflected in the following table:

**TABLE 5**

| Pigs immunised with peptide | Anti-3ABC antibodies |
|---|---|
| No. 1 | 0.277 |
| No. 2 | 0.127 |
| No. 3 | 0.142 |
| No. 4 | 0.206 |
| No. 5 | 1.108 |
| No. 6 | 1.174 |

An ELISA capable of detecting antibodies against non-structural proteins of the FMDV, can be used to discriminate between infected and non-infected animals, irrespective of whether or not they have been vaccined with an inactivated vaccine (Brocchi E. et al., "Comparative evaluation of six ELISAs for the detection of antibodies to the non-structural proteins of foot-and-mouth disease virus", Vaccine 2006). The results obtained show that this same principle can be applied to the immunisation using the dendrimeric peptide construct of the present invention, since the presence of antibodies was only detectable in the infected animals, but not in the protected ones (immunised with the dendrimeric peptide construct and subsequently challenged).

## Claims

1. A dendrimeric peptide construct of formula (I):
(X-B)n-L-T (I)
wherein
T is a peptide comprising the SEQ ID NO: 1 sequence;
B is a peptide comprising the SEQ. ID NO: 2 sequence;
X is an acyl group which is bound to the N-terminal end of peptide B; n is 3 or 4; and
L corresponds to a lysine core of formula (II) 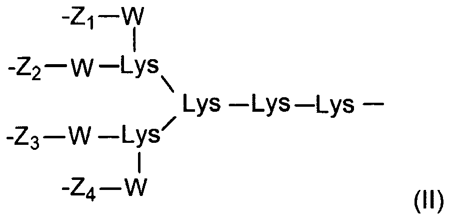 wherein
W is a linker group;
Z₁ -Z₄ are identical or different to each other and are selected from the group consisting of cysteine and -OH, provided that at least three of the Z₁ to Z₄ groups are cysteine;
the peptide B being bound to the lysine core by its C-terminal end and the peptide T being bound to said lysine core by its N-terminal end.

2. The dendrimeric peptide construct according to claim 1, wherein
T is a peptide of sequence SEQ ID NO: 1, and
B is a peptide of sequence SEQ ID NO: 2.

3. The dendrimeric peptide construct according to claim 1, wherein
T is a peptide comprising the sequence SEQ ID NO: 1, and
B is a peptide comprising the sequence SEQ ID NO: 5.

4. The dendrimeric peptide construct according to claim 1, wherein X is acetyl.

5. The dendrimeric peptide construct according to claim 1, wherein W is a group -(CH₂)ₙ-CO-, being n a number between 1 and 3.

6. The dendrimeric peptide construct according to claim 1, wherein L represents: 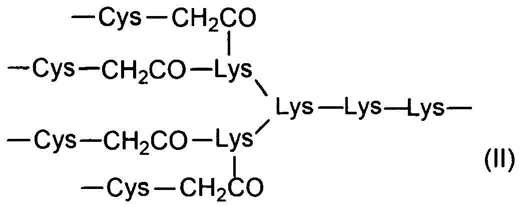 wherein each cysteine is bound to the CH₂CO linker group through its side chain by a thioether bond.

7. The dendrimeric peptide construct according to any of the preceding claims, which has the formula (III): 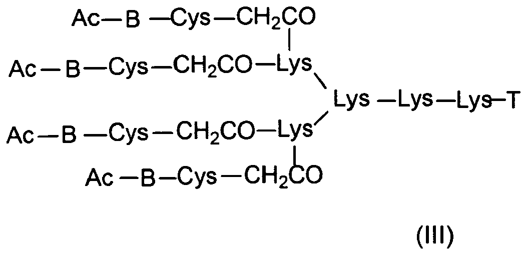 wherein
T is a peptide of SEQ ID NO: 1 sequence; and
B is a peptide of SEQ ID NO: 2 sequence.

8. A process for preparing a dendrimeric construct as defined in any of the claims 1-7, which comprises the ligation reaction of a peptide of formula (IV): 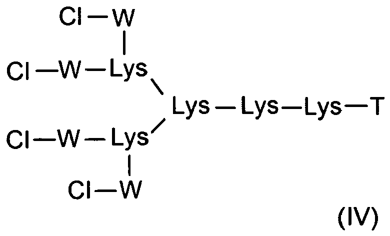 with a peptide (V),
X-B-Cys (V)
wherein
Cl is chlorine and
T, X, W and B are as defined in any of the preceding claims;
and wherein the ligation reaction is carried out in the presence of a chaotropic agent and at a pH between 8 and 9.

9. The process according to claim 8, wherein the reaction is carried out at a pH of 8.5,the chaotropic agent is guanidine hydrochloride, and the peptide of formula (V) is in a molar excess comprised between 3 and 5 in respect of the peptide in formula (IV).

10. A dendrimeric peptide construct according to any of claims 1-7 for use as an immunogen.

11. A veterinary composition that comprises an effective amount of at least one dendrimeric peptide construct as defined in any of claims 1-7.

12. The composition according to claim 11, which comprises adequate amounts of at least one adjuvant for its administration in the form of a vaccine.

13. A dendrimeric peptide construct defined in any of claims 1-7 for use in the prevention of foot and mouth disease in an animal.

## Patentansprüche

1. Dendrimeres Peptidkonstrukt der Formel (I):
(X-B)n-L-T (I)
wobei
T ein Peptid ist, das die Sequenz SEQ ID NO: 1 umfasst;
B ein Peptid ist, das die Sequenz SEQ ID NO: 2 umfasst;
X eine Acylgruppe ist, die mit dem N-Terminus des Peptids B verbunden ist; n 3 oder 4 ist; und
L einem Lysine-Kern der Formel (II) entspricht 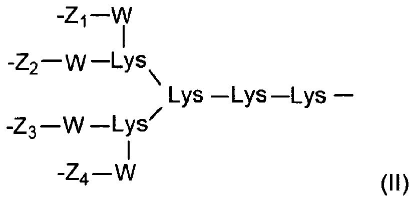 wobei
W eine Linkergruppe ist;
Z₁-Z₄ identisch oder verschieden voneinander und ausgewählt aus der Gruppe sind bestehend aus Cystein und -OH unter der Bedingung, dass mindestens drei der Z₁-Z₄ Gruppen Cystein sind;
wobei das Peptid B mit dem Lysine-Kern durch sein C-Terminus verbunden ist und das Peptid T mit dem Lysine-Kern durch sein N-Terminus verbunden ist.

2. Dendrimeres Peptidkonstrukt nach Anspruch 1, wobei
T ein Peptid mit der Sequenz SEQ ID NO: 1 ist, und
B ein Peptid mit der Sequenz SEQ ID NO: 2 ist.

3. Dendrimeres Peptidkonstrukt nach Anspruch 1, wobei
T ein Peptid ist, das die Sequenz SEQ ID NO: 1 umfasst, und
B ein Peptid ist, das die Sequenz SEQ ID NO: 5 umfasst.

4. Dendrimeres Peptidkonstrukt nach Anspruch 1, wobei X Acetyl ist.

5. Dendrimeres Peptidkonstrukt nach Anspruch 1, wobei W eine -(CH₂)ₙ-CO-Gruppe ist, wobei n eine Zahl zwischen 1 und 3 ist.

6. Dendrimeres Peptidkonstrukt nach Anspruch 1, wobei L für die folgende Struktur steht: 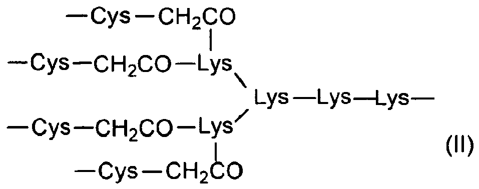 worin jedes Cystein mit der Linkergruppe CH₂CO durch seine Seitenkette mittels einer Thioetherbindung verbunden ist.

7. Dendrimeres Peptidkonstrukt nach einem der vorhergehenden Ansprüche, das die folgende Formel (III) hat: 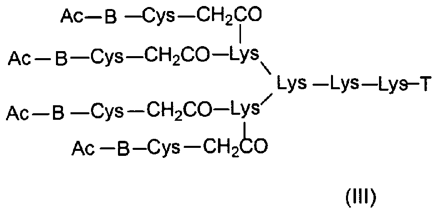 worin
T ein Peptid mit der Sequenz SEQ ID NO: 1 ist; und
B ein Peptid mit der Sequenz SEQ ID NO: 2 ist.

8. Verfahren zur Herstellung eines dendrimeren Konstrukts nach einem der Ansprüche 1 bis 7 umfassend die Ligationsreaktion eines Peptids der Formel (IV): 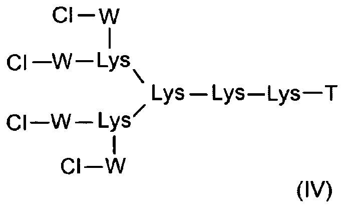 mit einem Peptid (V),
X-B-Cys (V)
wobei
Cl Chlor ist und T, X, W und B nach einem der vorhergehenden Ansprüche sind;
und wobei die Ligationsreaktion in Anwesenheit von einem chaotropen Mittel und bei einem pH-Wert zwischen 8 und 9 ausgeführt wird.

9. Verfahren nach Anspruch 8, wobei die Reaktion bei einem pH-Wert von 8,5 ausgeführt wird, das chaotrope Mittel Guanidinhypochlorid ist, und das Peptid der Formel (V) in einem molaren Überschuss zwischen 3 und 5 bezogen auf das Peptid der Formel (IV) vorhanden ist.

10. Dendrimeres Peptidkonstrukt nach einem der Ansprüche 1-7 zur Verwendung als Immunogen.

11. Tiermedizinische Verbindung umfassend eine wirksame Menge von mindestens einem dendrimeren Peptidkonstrukt so wie in einem der Ansprüche 1-7 definiert.

12. Verbindung nach Anspruch 11, die eine angemessene Menge von mindestens einem Hilfsmittel zur Verabreichung als Impfstoff enthält.

13. Dendrimeres Peptidkonstrukt so wie in einem der Ansprüche 1-7 definiert zur Verwendung bei der Prävention von Maul- und Klauenseuche bei Tieren.

## Revendications

1. Construction peptidique dendrimère de formule (I):
(X-B)n-L-T (I)
dans laquelle
T est un peptide comprenant la séquence SEQ ID NO: 1;
B est un peptide comprenant la séquence SEQ ID NO: 2;
X est un groupe acyle lié à l'extrémité N-terminale du peptide B; n est 3 ou 4; et
L correspond à un noyau de lysine de formule (II) 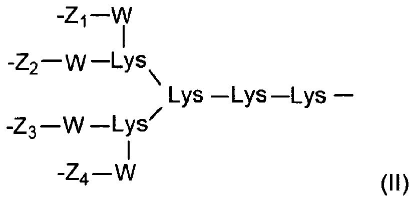 dans laquelle
W est un groupe de liaison;
Z₁-Z₄ sont identiques ou différents les uns des autres et ils sont choisis dans le groupe constitué de la cystéine et du groupe -OH à condition qu'au moins trois des groupes X₁-Z₄ soient des groupes cystéine;
étant le peptide B lié au noyau de lysine par son extrémité C-terminale et le peptide T lié audit noyau de lysine par son extrémité N-terminale.

2. Construction peptidique dendrimère selon la revendication 1 dans laquelle T est un peptide ayant la séquence SEQ ID NO: 1, et
B est un peptide ayant la séquence SEQ ID NO: 2.

3. Construction peptidique dendrimère selon la revendication 1 dans laquelle T est un peptide comprenant la séquence SEQ ID NO: 1, et
B est un peptide comprenant la séquence SEQ ID NO: 5.

4. Construction peptidique dendrimère selon la revendication 1 dans laquelle X est un groupe acétyle.

5. Construction peptidique dendrimère selon la revendication 1 dans laquelle W est un groupe -(CH₂)ₙ-CO- , étant n une chiffre allant de 1 à 3.

6. Construction peptidique dendrimère selon la revendication 1 dans laquelle L représente : 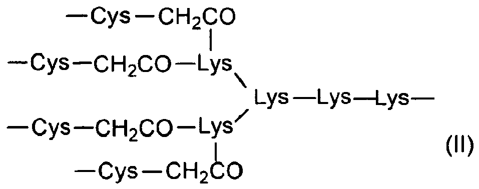 où chaque cystéine est liée au group de liaison CH₂CO par sa chaîne latérale par le biais d'une liaison thioéther.

7. Construction peptidique dendrimère selon l'une quelconque des revendications précédentes ayant la formule (III): 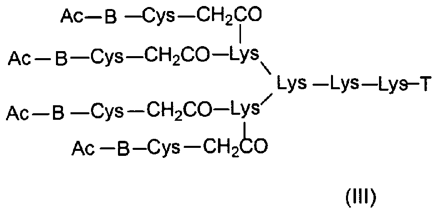 dans laquelle
T est un peptide ayant la séquence SEQ ID NO: 1; et
B est un peptide ayant la séquence SEQ ID NO: 2.

8. Procédé de préparation d'une construction dendrimère telle que définie dans l'une quelconque des revendications 1 à 7 comprenant la réaction de ligation d'un peptide de formule (IV): 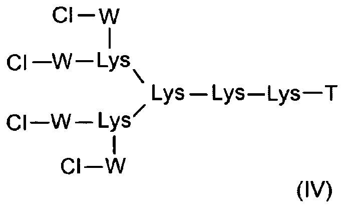 avec un peptide (V),
X-B-Cys (V)
dans laquelle CI est le chlore
T, X, W et B sont tels que définis dans une quelconque des revendications précédentes;
et dans lequel la réaction de ligation est effectuée en présence d'un agent chaotropique et à un pH compris entre 8 et 9.

9. Procédé selon la revendication 8, dans lequel la réaction est effectuée à un pH de 8,5, l'agent chaotropique est l'hydrochlorure de guanidine, et le peptide de formule (V) est en un excès molaire d'entre 3 et 5 par rapport au peptide de la formule (IV).

10. Construction peptidique dendrimère selon l'une quelconque des revendications 1 à 7 destinée à être utilisée en tant qu'immunogène.

11. Composition vétérinaire comprenant une quantité effective d'au moins une construction peptidique dendrimère telle que définie dans l'une quelconque des revendications 1 à 7.

12. Composition selon la revendication 11 qui comprend des quantités adéquates d'au moins un adjuvant destinée à une administration sous la forme de vaccin.

13. Construction peptidique dendrimère telle que définie dans l'une quelconque des revendications 1 à 7 destinée à être utilisée pour la prévention de la fièvre aphteuse chez les animaux.


### SEQUENCE LISTING

<110> Universitat Pompeu Fabra Consejo Superior de Investigaciones Cientificas Instituto Nacional de Investigación y Tecnología Agraria y Alimentaria
<120> Dendrimeric peptide construct for the prevention of foot-and-mouth virus in animals
<130> P774PC00
<150> ES P200602142
   <151> 2006-08-02
<160> 5
<170> PatentIn version 3.3
<210> 1
   <211> 15
   <212> PRT
   <213> Foot-and-mouth disease virus
<400> 1
   Ala Ala Ile Glu Phe Phe Glu Gly Met Val His Asp Ser Ile Lys
   1               5                   10                  15
<210> 2
   <211> 18
   <212> PRT
   <213> Foot-and-mouth disease virus
<400> 2
   Tyr Thr Ala Ser Ala Arg Gly Asp Leu Ala His Leu Thr Thr Thr His
   1               5                   10                  15
   Ala Arg
<210> 3
   <211> 16
   <212> PRT
   <213> Artificial
<220>
   <223> peptide derived from the 3A protein from the foot-and-nouth disease virus
<220>
   <221> MOD_RES
   <222> (16)..(16)
   <223> AMIDATION
<400> 3
   Leu Leu Ala Ala Ile Glu Phe Phe Glu Gly Met Val His Asp Ser Ile
   1               5                   10                  15
<210> 4
   <211> 19
   <212> PRT
   <213> Artificial
<220>
   <223> modified peptide of the VP1 protein from the foot-and-mouth disease virus
<220>
   <221> MOD_RES
   <222> (1)..(1)
   <223> ACETYLATION
<220>
   <221> MOD_RES
   <222> (19)..(19)
   <223> AMIDATION
<400> 4
   Tyr Thr Ala Ser Ala Arg Gly Asp Leu Ala His Leu Thr Thr Thr His
   1               5                   10                  15
   Ala Arg His
<210> 5
   <211> 19
   <212> PRT
   <213> Foot-and-mouth disease virus
<400> 5
   Pro Val Thr Asn Val Arg Gly Asp Leu Gln Val Leu Ala Gln Lys Ala
   1               5                   10                  15
   Ala Arg Thr